(19) Europäisches Patentamt
European Patent Office
Office européen des brevets

(11) EP 4 293 012 A1

(12) EUROPÄISCHE PATENTANMELDUNG

(43) Veröffentlichungstag:
**20.12.2023 Patentblatt 2023/51**

(21) Anmeldenummer: **22179609.7**

(22) Anmeldetag: **17.06.2022**

(51) Internationale Patentklassifikation (IPC):
***C07C 319/28*** (2006.01) ***C07C 319/20*** (2006.01)
***C07C 323/58*** (2006.01) ***C07K 5/062*** (2006.01)

(52) Gemeinsame Patentklassifikation (CPC):
(C-Sets verfügbar)
**C07C 319/28; C07C 319/20; C07K 5/0606** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
- **FEY, Karl-Georg**
  **63543 Neuberg (DE)**
- **DYBALLA, Claudia**
  **81825 München (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

# (54) VERFAHREN ZUR GEWINNUNG VON GEMISCHEN ENTHALTEND METHIONIN UND KALIUMHYDROGENCARBONAT

(57) Gegenstand der Erfindung ist ein Verfahren zur Gewinnung von Gemischen enthaltend Methionin und Kaliumhydrogencarbonat aus wässrigen Lösungen oder Suspensionen enthaltend titrierbares Kalium in Form von Kaliumhydrogencarbonat und/oder Kaliumcarbonat, Methionin, und
4,5 - 12,0 Gew. Methionyl-methionin, dadurch gekennzeichnet, dass man den eingesetzten Lösungen oder Suspensionen bei einer Temperatur von 15 - 60 °C $CO_2$ zuführt (carbonisiert), so dass ein Gemisch enthaltend Methionin und Kaliumhydrogencarbonat als Niederschlag ausfällt, der im Mittel max.6,5 Gew.% Met-Met enthält und dieser von der Mutterlauge abgetrennt wird.

EP 4 293 012 A1

(52) Gemeinsame Patentklassifikation (CPC): (Forts.)

C-Sets
**C07C 319/20, C07C 323/58;**
**C07C 319/28, C07C 323/58**

## Beschreibung

### Gebiet der Erfindung

Die vorliegende Erfindung betrifft ein Verfahren zur Gewinnung von Gemischen enthaltend Methionin und Kaliumhydrogencarbonat und gegebenenfalls Kaliumcarbonat aus wässrigen Lösungen oder Suspensionen enthaltend Methionin, Kaliumcarbonat, Kaliumhydrogencarbonat und Methionyl-methionin und dabei insbesondere eine Methode zur Rückgewinnung von Methionin und Kaliumhydrogencarbonat aus mit Met-Met angereicherten Methionin-Mutterlaugen.

### Hintergrund der Erfindung

Die Aminosäure Methionin wird auf vielen Gebieten angewendet, wie zum Beispiel für pharmazeutische, Gesundheits- und Fitnessprodukte, insbesondere jedoch als Futtermitteladditiv in vielen Futtermitteln für verschiedene Nutztiere. Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylmercaptopropanal (MMP, hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt und dieses anschließend alkalisch mit Alkalimetallhydroxid und/oder Alkalimetallcarbonat und Alkalimetallhydrogencarbonat, beispielsweise Kaliumhydroxid und/oder Kaliumcarbonat und Kaliumhydrogencarbonat, zum Alkalimethionat (z.B. Kaliummethioninat) hydrolysiert (siehe Formelschema 1). Methionin wird schließlich durch saure Behandlung z.B. mit Kohlendioxid (Carbonisierung)aus seinem Alkalimetallsalz freigesetzt (siehe Formalschema 2), welches als Präzipitat aus der Alkalimetallcarbonat und Alkalimetallhydrogencarbonat (z.B. Kaliumcarbonat und Kaliumhydrogencarbonat) enthaltenden Mutterlauge abfiltriert wird. Das Filtrat, die Alkalimetallhydrogencarbonat (z.B. Kaliumcarbonat und Kaliumhydrogencarbonat) enthaltende Mutterlauge (1. Mutterlauge), wird zur Verseifung von Methioninhydantoin im Kreislauf geführt basierend auf dem Degussa-Kaliumcarbonat-Kreislaufverfahren (siehe z.B. EP 780370 A2).

**Formelschema 1: Methioninhydantoinverseifung**

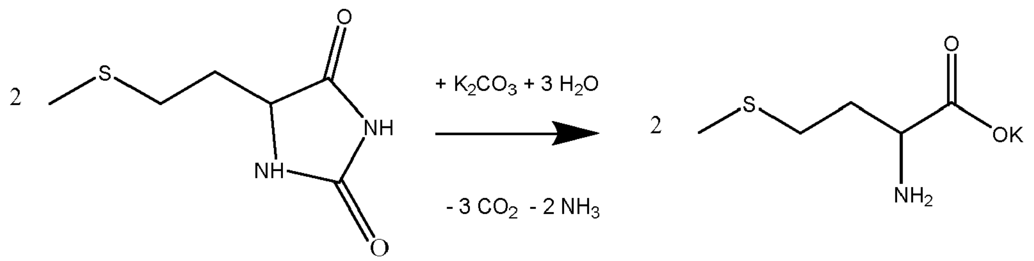


**Formelschema 2: Carbonisierung**

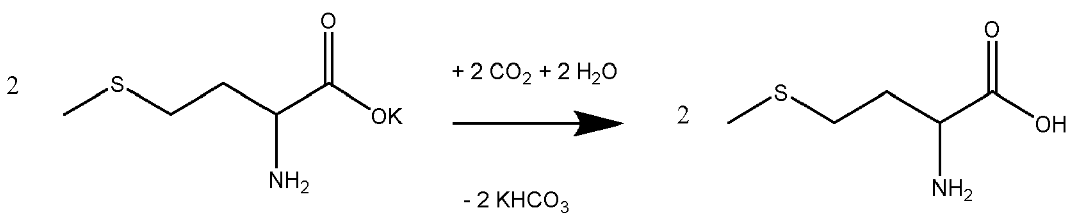


Wie bei allen Kreislaufprozessen bedarf auch dieser Prozess einer Ausschleusung der Mutterlauge, um die gebildeten Nebenprodukte nicht über das erträgliche Maß hinaus ansteigen zu lassen. Die ausgeschleuste Mutterlauge enthält jedoch noch das Produkt Methionin, Alkalimetallcarbonat und Alkalimetallhydrogencarbonat (z.B. Kaliumcarbonat und Kaliumhydrogencarbonat), das für die Verseifung von Methionin-Hydantoin nützlich ist. Um möglichst viel Methionin und Kaliumhydrogencarbonat aus der Mutterlauge zurückzugewinnen, kann man die auszuschleusende Mutterlauge einer zweiten Carbonisierung unterwerfen. Über die Gewinnung von Methionin und Kaliumhydrogencarbonat aus der 1. Mutterlauge durch eine zweite Carbonisierung gibt es verschiedene Prozesse, wie z. B. in DE2421167A1 und EP839804A2 berichtet wird. In der genannten EP-Schrift wird zur Fällung des Methionins ein wasserlösliches Lösungsmittel benötigt, was zusätzlichen Aufwand und mögliche Verunreinigungen bedeutet.

Eine weitere Gewinnung von Methionin und Kaliumhydrogencarbonat aus Mutterlaugen, wie z. B. aus der Mutterlauge der zweiten Carbonisierung (2.Mutterlauge), ist schwieriger. Dies liegt daran, dass Verunreinigungen in solchen Mutterlaugen deutlich mehr angereichert sind und diese Verunreinigungen nachfolgende Prozessschritte stören können. So offenbart EP1760074A1 eine Methode zur Rückgewinnung von Methionin und Kaliumhydrogencarbonat aus der Mutterlauge der 2. Carbonisierung durch eine 3. Carbonisierung. Das dabei ausgefallene Gemisch von Methionin und Kaliumhydrogencarbonat wird durch Filtration abgetrennt und kann direkt zur Hydantoin-Verseifung zurückgeführt werden. In dieser Methode lässt sich jedoch der Niederschlag der 3. Carbonisierung nicht gut filtrieren, wie es in EP2133328A2 und in EP2186798A1 berichtet wird. Der Grund liegt darin, dass sich das Nebenprodukt Methionylmethionin (Met-Met) in der Mutterlauge der zweiten Carbonisierung deutlich angereichert hat und Met-Met die Abiltrierbarkeit des Niederschlags der 3. Carbonisierung negativ beeinflusst. Aus diesem Grunde wird versucht, den Gehalt an Met-Met in der 2. Mutterlauge zu reduzieren. So wird in EP2133328A2 und in EP2186798A1 die Mutterlauge der 2. Carbonisierung zuerst aufkonzentriert und anschließend auf eine erhöhte Temperatur, z. B. auf 180°C, erhitzt. Durch diese Behandlung wird Met-Met in der 2. Mutterlauge teilweise zu Methionin gespalten. Die dadurch erhaltene Mutterlauge ist daraufhin geeignet für eine Rückgewinnung des darin enthaltenen Methionins und Kaliumhydrogencarbonats durch eine dritte Carbonisierung, wie z.B. in EP2133328A bzw. EP2186798A1 beschrieben oder durch die Rückführung der Mutterlauge zur 2. Carbonisierung, wie in EP2186797A1 beschrieben.

Eine alternative Methode zur Reduktion des Gehalts an Met-Met wird in EP2133329A2 offenbart. Nach EP2133329A entsteht in der Methionin-Hydantoin-Verseifung weniger Met-Met, wenn die Verseifung in einem nicht gerührten Reaktor stattfindet (keine Rückvermischung) und nach der Entfernung von $CO_2$ und Wasser die verbleibende Verseifungslösung in einem separaten Reaktor weiter erwärmt wird. Hier benötigt man also zwei separate Reaktoren und ein Erhitzen der basischen Lösung bei erhöhter Temperatur wird auch nicht vermieden was ebenfalls nachteilig ist. Ebenso nachteilig ist die Verwendung des Hilfsstoffes Polyvinylalkohol, was zusätzlichen Aufwand und Kosten sowie ggf. entsprechende Rückstände im Produkt verursacht.

Weiter ist bekannt aus EP1840119A2, dass die oben für eine Met-Met Spaltung notwendigen Bedingungen in der basischen Methionin-Mutterlauge (hohe Konzentration und Temperatur) sehr korrosiv wirken und deswegen teure Werkstoffe für die Reaktoren (wie z. B. Zirkonium oder Super-Duplex Stahl) benötigt werden. Darüber hinaus sind die o. g. Bedingungen für die Spaltung von Met-Met zu Methionin sehr harsch. Diese Bedingungen können ebenfalls zur Zersetzung von Methionin führen, was auch von Nachteil ist, genauso wie der erhöhte Energiebedarf für diesen zusätzlichen Prozessschritt.

Daher wurden neue Methoden zur Rückgewinnung von Methionin und Kaliumhydrogencarbonat aus mit Met-Met angereicherten Methionin-Mutterlaugen, wie z. B. der Mutterlauge der 2. Carbonisierung, benötigt welche die Nachteile der im Stand der Technik beschriebenen Methoden nicht oder wenigstens nur in verringertem Maß aufweisen.

Der Prozess zur Rückgewinnung von Wertstoffen aus der ausgeschleusten Mutterlauge durch Carbonisierung und Abtrennung hat eine zentrale Aufgabe: Die Trennung des bei der Carbonisierung erzeugten Niederschlages (er enthält v.a. die Wertstoffe Kaliumhydrogencarbonat und Methionin) und der Mutterlauge (sie ist reich an Nebenkomponenten wie Formiat und Met-Met). Der Niederschlag wird zurück in den Prozess geführt, die verbleibende Mutterlauge (z.B. die 2. oder 3. Mutterlauge) stellt das Abfallprodukt dar.

Wenn diese Fest-Flüssig-Trennung nicht gut funktioniert, d.h. wenn der Niederschlag schlecht abtrennbar, z.B. schlecht abfiltrierbar ist und daher nur mit viel Mutterlauge behaftet in den Prozess zurückgeführt werden kann, dann hat dies zur Folge, dass auch viele Nebenkomponenten wieder zurück zum Hydrolyseschritt - der Hydantoin-Verseifung - gelangen, diese sich also im Prozess "aufschaukeln" und folglich wiederum mehr 1. Mutterlauge ausgeschleust werden muss, um diese Nebenkomponenten erneut aus dem Kreislauf zu entfernen. Die Abfiltrierbarkeit des Niederschlages bestimmt daher maßgeblich den Durchsatz durch die gesamte Aufarbeitung der ausgeschleusten 1. Mutterlauge. Die Filtrierbarkeit ist daher für die Wirtschaftlichkeit der gesamten Aufarbeitung maßgeblich.

Bei der Rückgewinnung von Methionin und Kaliumhydrogencarbonat aus mit Met-Met angereicherten Methionin-Mutterlaugen ist, wie aus den o.g. Patentschriften hervorgeht, nun gerade die Filtration problematisch. Laut den o.g. Schriften liegt dies an der höheren Konzentration von Met-Met in der 2. Mutterlauge. Aus diesem Grund werden dort auch mehrere Verfahren beschrieben, wie der Met-Met Gehalt in der Mutterlauge reduziert werden kann, um die Abfiltrierbarkeit des Niederschlages zu verbessern.

So wird die 2. Mutterlauge vor der Carbonisierung und Filtration z.B. erst für eine Dauer von 0,3 bis 10 Stunden (EP2186797A1) auf hohe Temperaturen (z.B. 160 - 180 °C) erhitzt, um Met-Met zu Methionin zu spalten. Dies erleichtert die folgende Filtration.

Die beschriebenen Lösungen haben jedoch den Nachteil, dass teures zusätzliches Equipment benötigt wird (z.B. ein korrosionsbeständiger Reaktor mit ausreichend Verweilzeit sowie Wärmeübertrager), ein zusätzlicher Energieeinsatz nötig ist und zudem auch der Wertstoff Methionin teilweise wieder abgebaut wird. Hierdurch entstehen erneut Nebenkomponenten.

**Aufgabe**

Es war daher Aufgabe der zugrundeliegenden Erfindung, ein Verfahren zur Rückgewinnung von Gemischen enthaltend Methionin, Kaliumhydrogencarbonat und ggf. Kaliumcarbonat aus wässrigen Lösungen oder Suspensionen enthaltend Methionin, ggf. Kaliumcarbonat, Kaliumhydrogencarbonat und Methionyl-methionin, insbesondere aus Mutterlaugen der Methioninherstellung, in der Methionyl-methionin nennenswert angereichert ist, bereitzustellen, bei dem vorhandenes Methionin, Kaliumhydrogencarbonat und ggf. Kaliumcarbonat in einem möglichst hohen Anteil als Niederschlag aus der Mutterlauge ausfällt und dieser möglichst gut abfiltriert werden kann, also eine möglichst gute Filtrierbarkeit aufweist.

Eine weitere damit in unmittelbarem Zusammenhang stehende Aufgabe war es, ein verbessertes Verfahren zur Herstellung von Methionin bereitzustellen, bei dem eine geringere Menge an Abfallprodukten anfällt bzw. entsorgt werden muss.

**Beschreibung der Erfindung**

Die vorliegende Erfindung löst das Problem der schlechten Abfiltrierbarkeit des bei der Carbonisieriung von mit Met-Met angereicherten Mutterlaugen anfallenden Niederschlags auf eine andere Weise als der Stand der Technik. Der Niederschlag der Carbonisierung enthält erfindungsgemäß die Stoffe Methionin, Kaliumhydrogencarbonat und ggf. in kleineren Anteilen Kaliumcarbonat und die zugehörige Mutterlauge darüber hinaus noch nennenswerte Mengen Methionyl-methionin. Die der Erfindung zugrundeliegende Erkenntnis liegt darin, dass das angereicherte Met-Met die Filtration nicht nennenswert stört, solange der ausgefallene, also im Niederschlag vorhandene, Gehalt an Methionylmethionin einen kritischen Wert nicht überschreitet, es also weitgehend in der Mutterlauge gelöst bleibt. Die Filtration wird erst dann schwierig, wenn Met-Met während der Carbonisierung in nennenswerten Mengen ausfällt und somit als Kristall im Niederschlag vorliegt.

Die o.g. Aufgabe wird demgemäß gelöst durch die Bereitstellung eines Verfahrens zur Gewinnung von Gemischen enthaltend Methionin, Kaliumhydrogencarbonat und ggf. Kaliumcarbonat aus wässrigen Lösungen oder Suspensionen, enthaltend

vorzugsweise 6,0 - 18,0 Gew.% titrierbares Kalium in Form von Kaliumhydrogencarbonat und/oder Kaliumcarbonat,
vorzugsweise 2,5 - 8,0 Gew.% Methionin, und
4,50 - 12,0 Gew.% Methionyl-methionin, dadurch gekennzeichnet, dass man den eingesetzten Lösungen oder Suspensionen bei einer Temperatur von 15 - 60 °C $CO_2$ zuführt (carbonisiert), so dass ein Gemisch enthaltend Methionin, Kaliumhydrogencarbonat und ggf. Kaliumcarbonat als Niederschlag ausfällt, der im Mittel max. 6,5 Gew.%, vorzugsweise max. 0,01 bis 5,0 Gew.% insbesondere max. 0,01 bis 3,0 Gew.% Met-Met enthält. Der angestrebte Met-Met-Gehalt kann dabei z.B. per HPLC fortlaufend an Proben aus dem abgetrennten Niederschlag bestimmt werden. So werden besonders gute Filtrierbarkeiten erreicht und damit die oben genannten Nachteile der Verfahren aus dem Stand der Technik überwunden. Die eingesetzten wässrigen Lösungen oder Suspensionen weisen einen alkalischen pH-Wert von ca. 11 bis 12 auf, der durch die Carbonisierung abgesenkt wird, vorzugsweise auf einen pH-Wert von 7,8 bis 9,5, besonders bevorzugt von 8,3 bis 9,5, insbesondere von 8,4 bis 9,5 und ganz besonders bevorzugt von 8,4 bis 9,0, gemessen mit einer Glaselektrode bei der jeweils eingestellten Temperatur. Der Met-Met-Gehalt im Niederschlag ist dabei auch deutlich abhängig vom pH-Wert in der carbonisierten Mutterlauge, so dass dieser auch zur Steuerung des Met-Met-Gehaltes im Niederschlag herangezogen werden kann.

Die Abtrennung des Niederschlags kann dabei z.B. durch Filtration oder Zentrifugation, insbesondere durch entsprechende dem Fachmann bekannte auch großtechnisch einsetzbare Aggregate erfolgen, die vorzugsweise kontinuierlich oder halbkontinuierlich betrieben werden, so dass die industriell üblichen Produktdurchsätze gefahren werden können. Zur Kontrolle und Steuerung des pH-Wertes und damit auch indirekt des technischen Effektes der guten Filtrierbarkeit können entweder regelmäßig Proben genommen und per Glaselektrode vermessen werden (vgl. Beispiele) oder man wählt eine kontinuierliche pH-Messung, so dass der gewünschte pH-Wert und damit die Filtrierbarkeit sehr einfach eingestellt werden können. Zur direkten Kontrolle und Steuerung des Met-Met-Gehaltes und damit auch des technischen Effektes der guten Filtrierbarkeit können entweder regelmäßig Proben aus der Mutterlauge genommen und per HPLC vermessen werden (vgl. Beispiele). Die HPLC-Bestimmung des erfindungsgemäß vorteilhaften Met-Met-Gehaltes kann aber auch automatisiert von statten gehen

Bevorzugt wird dabei ein Verfahren, das dadurch gekennzeichnet ist, dass man die eingesetzten Lösungen oder Suspensionen ggf. durch Aufkonzentrieren auf einen Gehalt von

6,0 - 18 Gew.% titrierbares Kalium in Form von Kaliumcarbonat und/oder Kaliumhydrogencarbonat,
2,5 - 8,0 Gew.% Methionin und

4,5 - 12,0 Gew.% Methionyl-Methionin bringt und dann

bei einer Temperatur von 15 - 60 °C, vorzugsweise 25 - 55 °C solange $CO_2$ zuführt (carbonisiert) bis ein pH-Wert von 7,8 bis 9,5, vorzugsweise 8,3 bis 9,5, besonders bevorzugt von 8,4 bis 9,5, insbesondere von 8,4 bis 9,0 erreicht wird, gemessen mit einer Glaselektrode bei der eingestellten Temperatur, so dass ein Gemisch enthaltend Methionin, Kaliumhydrogencarbonat und ggf. Kaliumcarbonat als Niederschlag ausfällt und dieser von der Mutterlauge abgetrennt wird, bei dem der Niederschlag im Mittel max. 6,5 Gew.%, vorzugsweise max. 0,01 bis 5,0 Gew.%, insbesondere max. 0,01 bis 3,0 Gew.% Met-Met enthält.

Erfindungsgemäß wird somit ein Verfahren bereitgestellt, bei dem die Rückgewinnung von Methionin und Kaliumhydrogencarbonat aus mit Met-Met angereicherten Methionin-Mutterlaugen erreicht werden kann, indem die Prozessparameter (insbesondere der Carbonisierung) so gewählt werden, dass während der Carbonisierung möglichst viel Methionin und Kaliumhydrogencarbonat ausfallen und gleichzeitig das Met-Met aber im ausgefallenen Niederschlag einen kritischen Wert nicht überschreitet, also größtenteils in der Mutterlauge gelöst bleibt.

In der vorliegenden Erfindung ist eine Spaltung von Met-Met vor der Carbonisierung nicht mehr notwendig, wodurch erheblich Energie und Kosten eingespart werden können. Durch das erfindungsgemäße Verfahren in seinen vorstehend genannten Varianten kann sogar eine deutliche Verbesserung der Filtrierbarkeit des Niederschlags aus der Carbonisierung erreicht werden. Das zeigt sich z.B. an dem deutlich geringeren Filtrationswiderstand von $1,2*10^6$ m/kg im erfindungsgemäßen Beispiel 1 gegenüber dem in EP2186797A1 Beispiel 1 (2.Kristallisierung) beschriebenen Wert von $1,6*10^9$ m/kg bzw. gegenüber dem in EP2133328A2 Beispiel 1 (3.Kristallisierung) beschriebenen Wert von $0,64*10^{10}$ m/kg.

Erfindungsgemäß wird insbesondere die 2. Mutterlauge der 2. Carbonisierung zunächst aufkonzentriert. Der Druck wird dabei niedrig gewählt (z.B. 300 mbara) um die Mutterlauge schonend bei geringer Temperatur aufzukonzentrieren. Dadurch wird die Spaltung von Methionin und Met-Met vermieden. Die Mutterlauge wird dabei z.B. um den Faktor 2 aufkonzentriert. Die Konzentration an freiem Kalium liegt dann z.B. bei 13,4 Gew.% und die Konzentration von Methionin liegt z.B bei 4,9 Gew.%. Auch Met-Met wurde nun angereichert, die Konzentration liegt z.B. bei 7 Gew.% (siehe Beispiel 1). Anschließend wird diese Lösung unter Zugabe von $CO_2$ carbonisiert. Der Druck liegt dabei im Bereich 1 - 6 bara, vorzugsweise 1,5 - 2,5 bara, um ein Schäumen der Suspension bei deren Entspannung zu reduzieren. Die Temperatur liegt zwischen 15 - 60 °C vorzugsweise 25 - 35 °C, um den Einsatz von Kaltwasser zu vermeiden und stattdessen das energetisch günstigere Kühlwasser verwenden zu können. Die Verweilzeit in der $CO_2$-Zufuhr liegt zwischen 20 und 180 Minuten, vorzugsweise bei etwa 60 Minuten. Im Reaktor wird der pH-Wert der Suspension mit einer Glaselektrode bei der jeweiligen Temperatur gemessen. Dieser wird über die $CO_2$-Zugabe eingestellt. Die aufkonzentrierte Mutterlauge hat einen pH-Wert von etwa 11. Die Mutterlauge im Reaktor wird nun nicht vollständig (d.h. bis zum Gleichgewicht beim jeweiligen Druck) carbonisiert. Es wird hingegen nur so viel $CO_2$ zugegeben, bis der pH-Wert einen Wert von 7,8 bis 9,5 erreicht hat, z.B. 8,5.

Die Erfinder haben festgestellt, dass die Löslichkeit von Met-Met in dieser Matrix insbesondere von Temperatur und pH abhängig ist. Der Ziel-pH-Wert im Reaktor (und damit die $CO_2$ Dosierung) wird dabei vorteilhafterweise so gewählt, dass nur ein geringer Anteil an Met-Met nämlich max. 6,5 Gew.%, vorzugsweise max. 0,1 bis 5,0 Gew.%, besonders bevorzugt max.0,1 bis 3,0 Gew.% im Niederschlag vorliegt. Der Met-Met-Gehalt wird dabei üblicherweise per HPLC an zunächst filterfeuchten, anschließend mit Aceton gewaschenen und getrockneten Proben des Niederschlags bestimmt. Dabei handelt es sich naturgemäß um einen Querschnittswert aus dem kristallinen Met-Met-Anteil im Niederschlag und dem Met-Met-Anteil

in der am Niederschlag anhaftenden Mutterlauge. Dieser Summenwert bzw. Mittelwert ist aber auch indirekt ein Maßstab für den eigentlich relevanten kristallinen Anteil von Met-Met, so dass er als Stellgröße zur Einstellung der Filtrierbarkeit herangezogen werden kann.

Der bevorzugte Ziel-pH-Wert ist daher abhängig von:

- Konzentration an Met-Met im Filtrat der 2. Carbonisierung
- Konzentration von Kalium, Methionin und Met-Met nach der Aufkonzentration, welche jeweils mit dem Aufkonzentrierungsfaktor korreliert, der bei typischerweise1,5 bis 2,5 liegt
- Temperatur im Reaktor

Nach der Carbonisierung wird der Niederschlag typischerweise abfiltriert. Da die Mutterlauge nicht bis zum Gleichgewicht carbonisiert wurde, enthält der Niederschlag neben Methionin und Kaliumhydrogencarbonat nur kleinere Mengen an Met-Met. Der Niederschlag wird wieder dem Prozess zugeführt, vorzugsweise vor der Hydantoin-Verseifung (Reaktionsschritt umfassend die Hydrolyse von 5-[2-(Methylthio)ethyl]imidazolidin-2,4-dion). Das darin evtl. enthaltene Met-Met wird in der Hydantoin-Verseifung zu Methionin gespalten. Dies erhöht genau wie die Rückführung des im Niederschlag enthaltenen Methionins die Ausbeute des Methionin-Verfahrens. Das Filtrat ist durch die Aufkonzentrierung ca. um den Faktor 2 reicher an Nebenkomponenten und wird vorzugsweise entsorgt. Durch die Aufkonzentrierung um

ca. Faktor 2 ist auch die Menge des Filtrates um ca. Faktor 2 geringer. Dadurch wird der flüssige Abfallstrom des Verfahrens deutlich reduziert.

Das Verfahren wird vorteilhafterweise so geführt, dass der Druck dabei im Bereich 1 - 6 bara, vorzugsweise 1,5 - 2,5 bara liegt.

Die typischen Verweilzeiten liegen zwischen 20 und 180 Minuten, vorzugsweise bei etwa 60 Minuten.

Zum Einsatz als wässrige Lösungen oder Suspensionen enthaltend Methionin, Kaliumcarbonat, Kaliumhydrogencarbonat und Methionyl-methionin in dem erfindungsgemäßen Verfahren wird bevorzugt eine Mutterlauge aus der Isolierung von Methionin im Verfahren zur Herstellung von Methionin via alkalische Methioninhydantoinverseifung eingesetzt, wie es im Grundsatz z.B. in der EP 780370 A2 beschrieben ist.

Vorzugsweise handelt es sich bei der Mutterlauge um die Mutterlauge der Filtration nach wiederholter Carbonisierung, insbesondere der 3.Carbonisierung, weil hier der Vorteil der Rückgewinnung ansonsten verlorener Wertstoffe am größten ist.

Gegenstand der Erfindung ist auch ein Gemisch enthaltend Methionin, Kaliumhydrogencarbonat und ggf. Kaliumcarbonat hergestellt gemäß dem vorstehend beschriebenen Verfahren sowie ein Gemisch enthaltend Methionin, $KHCO_3$ und Met-Met, wobei der Met-Met Gehalt bei max.6,5Gew.% liegt, wie es auch nach dem erfindungsgemäßen Verfahren hergestellt werden kann.

Ebenso Gegenstand der Erfindung ist die Verwendung solcher Gemische zur Herstellung von Methionin, insbesondere als Verseifungsagenz und zusätzliche Methioninquelle. Dies hat den großen Vorteil der Steigung der Verfahrensökonomie durch Ressourcenschonung und Erhöhung der Ausbeuten, was bei einer typischen Anlagenleistung von ca.100.000 Tonnen Methionin pro Jahr signifikant zu Buche schlägt.

Ein weiterer Gegenstand der Erfindung ist ein Gesamtverfahren zur Herstellung von Methionin, das die folgenden Schritte (1) bis (6) umfasst (siehe auch Figur 1, Blockschema mit prinzipiellem Verfahrensablauf):

(1) einen Reaktionsschritt, umfassend die Hydrolyse von 5-[2-(Methylthio)ethyl]imidazolidin-2,4-dion in Gegenwart einer basischen Kaliumverbindung zu einem Kaliummethioninat und Kaliummethionylmethioninat enthaltenden Hydrolysat;

(2) einen ersten Kristallisationsschritt, der die Einführung von Kohlendioxid (1.Carbonisierung) in das in Schritt (1) erhaltene Hydrolysat umfasst, um eine Suspension enthaltend Methionin, Methionyl-methionin, Kaliumhydrogencarbonat und ggf. Kaliumcarbonat zu erzeugen und dabei Methionin auszufällen, und Trennen der Suspension in einen Methionin enthaltenden 1.Niederschlag und eine Methionyl-methionin enthaltende 1.Mutterlauge;

(3) das Konzentrieren der in Schritt (2) erhaltenen 1.Mutterlauge (1.Konzentrationsschritt),

(4) Rückführung eines ersten Teils der konzentrierten 1. Mutterlauge aus (3) in den Reaktionsschritt (1)

(5) einen zweiten Kristallisationsschritt, der das Einleiten von Kohlendioxid in den zweiten Teil der konzentrierten 1. Mutterlauge aus (3) (2.Carbonisierung) umfasst, um Methionin und Kaliumhydrogencarbonat auszufällen, und das Trennen der resultierenden Suspension in einen 2.Niederschlag und eine 2.Mutterlauge;

(6) einen dritten Kristallisationsschritt, der das Konzentrieren der in Schritt (3) erhaltenen 2.Mutterlauge, das Einleiten von Kohlendioxid in die konzentrierte 2.Mutterlauge umfasst, um Methionin und Kaliumhydrogencarbonat auszufällen, und die Trennung der resultierenden Suspension in einen ein Gemisch aus Methionin, Kaliumhydrogencarbonat und gegebenenfalls Kaliumcarbonat enthaltenden 3.Niederschlag und eine Methionylmethionin enthaltende 3.Mutterlauge gemäß dem vorstehend beschriebenen Verfahren, so dass der 3.Niederschlag im Durchschnitt max. 6,5 Gew.%, vorzugsweise max. 0,01 bis 5 Gew.%, besonders bevorzugt max.0,01 bis 3 Gew.% Met-Met enthält.

Als basische Kaliumverbindung wird vorzugsweise Kaliumhydroxid, Kaliumcarbonat und/oder Kaliumhydrogencarbonat verwendet sowie zumindest ergänzend die vorstehend genannten Kaliumhydrogencarbonat und ggf. Kaliumcarbonat enthaltenden Niederschläge aus den Carbonisierungsstufen.

Durch die Bereitstellung des erfindungsgemäßen Gesamtverfahrens ist es gelungen, die Aufgabe zu lösen, ein verbessertes Verfahren zur Herstellung von Methionin bereitzustellen, bei dem die Ausbeute an Methionin erhöht wird, mehr basisches Kalium aus der Mutterlauge rezykliert wird und somit eine deutlich geringere Menge an Abfallprodukten anfällt bzw. entsorgt werden muss. Dies ist vor dem Hintergrund der typischen Produktionsleistung von ca. 100.000 Tonnen pro Jahr einer typischen Methioninanlage, insbesondere im Hinblick auf den Vorteil der Ressourcenschonung, von einem hohen ökonomischen und ökologischen Wert.

Des Weiteren bevorzugt ist ein Verfahren, bei dem man den 3. Niederschlag in den Hydrolyseschritt (1) oder in einen der weiteren Schritte (2) bis (4) zurückführt, wo er aufgrund seines hohen Kaliumgehaltes dazu dient, den Ersatzbedarf an Kalium zu reduzieren sowie das enthaltene Methionin zurückzuführen.

Außerdem vorteilhaft ist es, wenn man die 1. Mutterlauge und den 3. Niederschlag vereinigt und anschließend in den Hydrolyseschritt (1) oder den Konzentrationsschritt (3) zurückführt, was den Vorteil hat, dass es die Komplexität des Verfahrens herabsetzt.

Die bei dem Verfahren als Abfallstoff erhaltene 3.Mutterlauge kann sowohl einfach entsorgt oder aber einer

weiteren Isolierung von Wertstoffen zugeführt werden. Diese sind insbesondere das im Kreislauf gefahrene Kaliumhydrogencarbonat sowie das Verfahrensendprodukt Methionin.

**Beispiele**

**Verwendete Methoden**

**1. High Performance Liquid Chromatography (HPLC)**

Die chromatographischen Untersuchungen (Methionin und Met-Met) wurden mittels HPLC der Firma JASCO an einer geeigneten RP-Säule mit anschließender UV-Detektion bei 210 nm durchgeführt. Als Laufmittel diente ein phosphorsaures Acetonitril-Wasser-Gemisch. Bei einem Fluss von 1 mL/min wurden 10 $\mu$L der jeweiligen Probelösung injiziert. Die Kalibrierung erfolgte im Vorfeld durch die Injektion geeigneter Kalibrierlösungen entsprechender Referenzverbindungen aus dem Methionin- bzw. Met-Met-Verfahren, die Auswertung erfolgte durch Peakflächenvergleich mittels der externen Standardmethode. Die Durchführung der Standardmethode ist dem Fachmann bekannt.

**Probengewinnung zur Bestimmung von Met-Met im Niederschlag:**

Ein Raumteil des abfiltrierten Niederschlags aus der Carbonisierung wurde auf einer Laborfilternutsche gesammelt und mit zwei Volumenequivalenten Aceton übergossen und mit Hilfe eines reduzierten Drucks im Wasserstrahlvakuum abgesaugt. Dabei wurde der noch vorhandene und in Aceton etwas lösliche Anteil der am Niederschlag anhaftenden Mutterlauge weitgehend entfernt. Es wurde noch für 5 min weitergesaugt und dann in einem Trockenschrank bis zur Gewichtskonstanz getrocknet, so dass das restliche Aceton entfernt war. Anschließend wurde ein geeigneter Anteil des so vorbehandelten Niederschlags eingewogen, im HPLC-Laufmittel aufgenommen und mit weiterem HPLC-Laufmittel auf eine geeignete Konzentration herunterverdünnt und die so erhaltene Probenlösung wie oben erläutert in die HPLC injiziert.

**2. Kaliumtitration**

Alkalische Kalium-Salze, wie Kaliumcarbonat, Kaliumhydrogenkarbonat, Kalium-Methioninat sind in fast allen Prozesslösungen des Methionin-Prozesses enthalten und werden hier unter dem Begriff "titrierbares Kalium" zusammengefasst. Es erfolgte eine titrimetrische Bestimmung von "titrierbarem Kalium" in wässriger Lösung, insbesondere in Prozesslösungen als Summenparameter. Diese erfolgt als potentiometrische Titration mit 0,1 Molarer HCL an den betreffenden, Kalium enthaltenden alkalischen wässrigen Prozesslösungen bis pH 4,6. Bei der Säure/Base-Titration wird der Säure-Verbrauch als "titrierbares Kalium" angegeben.
1 ml 0,1 Molarer HCL entspricht dabei einem Gewichtsequivalent von 3,91mg $K^+$ entsprechend beispielsweise der Reaktion mit $KHCO_3$:

$$KHCO_3 + HCl \rightarrow KCl + H_2O + CO_2$$

**Anmerkung:** Wenn im Text der Begriff Equivalent (Eq oder eq) verwendet wird, ist damit Molequivalente (auch Moleq oder moleq) gemeint.

**3. Messung des Kuchenwiderstands als Maß für die Filtrierbarkeit einer Suspension**

Der sogenannte Kuchenwiderstand wurde gemäß der VDI-Richtlinie VDI 2762 Blatt 2, Dezember 2010 bestimmt. Der Kuchenwiderstand wird in $1/m^2$ angegeben. Der flächenbezogene Kuchenwiderstand $\alpha_H$ wird in $1/m^2$ angegeben, der massenbezogene Kuchenwiderstand $\alpha_M$ wird in m/kg angegeben. Die Umrechnung der beiden Größen erfolgt über die Dichte des trockenen Filterkuchens ($\rho_s$ in $kg/m^3$): $\alpha_H = \alpha_M * \rho_s$ Je höher der Wert des Kuchenwiderstands, desto schlechter ist die Filtrierbarkeit. Darüber hinaus wurde die Filtrierbarkeit auch direkt durch Beobachtung und Vergleich verschiedener Filtrationsversuche empirisch ermittelt. Dabei wurden folgende Filtrierbarkeitsnoten vergeben: Sehr gut - gut - akzeptabel - schlecht - sehr schlecht. Erfinderische Beispiele weisen demnach jeweils die Noten "sehr gut", "gut" oder "akzeptabel" auf.

**4. Herstellung von Methioninhydantoinverseifungslösung im Wesentlichen gemäß EP 780370 A2 als Ausgangslösung für die Beispiele**

Zunächst wurde im Wesentlichen gemäß EP 780370 A2, Beispiel 1 (Seite 10) aus MMP, HCN sowie Ammoniak

und $CO_2$ in Form von Ammoniumcarbonatlösung eine Methionin-Hydantoinlösung hergestellt und daraus durch Verseifung mit Hilfe von einer wässrigen, titrierbares Kalium, insbesondere in Form von $K_2CO_3$, enthaltenden Lösung im Wesentlichen gemäß Beispiel 6 der EP 780370 A2 Methioninhydantoinverseifungslösung (Hydrolysat) hergestellt. Dieses Hydrolysat, eine insbesondere Kaliummethioninat und Kalium-methionylmethioninat enthaltende alkalische Lösung, wurde mit Hilfe von $CO_2$ annähernd neutralisiert (1.Carbonisierung) analog zu Beispiel 7 der EP 780370 A2. Der dabei ausgefallene v.a. Methionin enthaltende 1.Niederschlag wurde wie ebenda beschrieben von der zugehörigen 1.Mutterlauge (1.Mula) abfiltriert.

Die 1. Mula wurde ein weiteres Mal carbonisiert (2.Carbonisierung) und der dabei erhaltene Niederschlag von der übrigbleibenden 2.Mula abfiltriert. Die so erhaltene 2.Mula wurde in den Beispielen 1 bis 12 als Ausgangslösung 1, 2 bzw. 3 mit der in Tab.1 jeweils angegebenen Zusammensetzung weiterverarbeitet.

Diese jeweilige Ausgangslösung (2. Mula) wurde mit einem Faktor von 1,6 bis 1,9 aufkonzentriert und die so erhaltene aufkonzentrierte 2.Mula jeweils einer 3.Carbonisierung zugeführt. Der dabei gebildete Niederschlag wurde jeweils über eine Filtrationseinheit abfiltriert und analysiert auf titrierbares Kalium K+, Methionin und Met-Met. Dabei wurde die Filtrierbarkeit beurteilt und z.T. auch der Kuchenwiderstand bestimmt (vgl. Tab.1).

In Tabelle 1 sind die Beispiele mit den Ergebnissen der Carbonisierungen und Filtrationen im Überblick aufgeführt, um die Abhängigkeit der Filtration von der Met-Met-Konzentration im Filterkuchen zu verdeutlichen:

**Tab. 1: Übersicht der eingestellten Parameter und Ergebnisse der Beispiele 1 bis 12**

| Beispiel | Verfahrensstufe | Konzentrationen in Gew.% | | | T in°C Carbonisierung | pH Carbo-nisierung | Filtrierbarkeit | Kuchenwiderstand $\alpha_M$ in m/kg |
|---|---|---|---|---|---|---|---|---|
| | | $K^+$ | Met | Met - Met | | | | |
| | 2. Mula (Ausgangslösung 1 für die Beispiele 1 und 2) | 7,0 | 2,6 | 3,7 | | | | |
| | 2. Mula nach Aufkonzentrierung um Faktor 1,9 | 13,4 | 4,9 | 7 | | | | |
| | | | | | | | | |
| 1 | 3. Mula nach Carbonisierung | 8,0 | 3,5 | 7,8 | 30 | 8,7 | | |
| | 3. Niederschlag u anhaftende Mula | 26,7 | 10 | 2,6 | | | sehr gut | $1,2*10^6$ *) |
| | | | | | | | | |
| 2 | 3. Mula nach Carbonisierung | 6,9 | 2,8 | 6,5 | 30 | 8,2 | | |
| | 3. Niederschlag u. anhaftende Mula | 27,3 | 11,3 | 6,4 | | | akzeptabel | $2,3*10^9$ *) |
| | | | | | | | | |
| | 2. Mula (Ausgangslösung 2 für die Beispiele 3 bis 5) | 7,3 | 2,6 | 3,3 | | | | |
| | 2. Mula nach Aufkonzentrierung um Faktor 1,8 | 13,1 | 4,6 | 5,9 | | | | |
| | | | | | | | | |
| 3 | 3. Mula nach Carbonisierung | 8,2 | 3,4 | 6,1 | 30 | 8,9 | | |
| | 3. Niederschlag u. anhaftende Mula | 33,2 | 8,7 | 1,2 | | | sehr gut | |
| | | | | | | | | |
| 4 | 3. Mula nach Carbonisierung | 7,6 | 2,6 | 6,2 | 30 | 8,5 | | |
| | 3. Niederschlag u. anhaftende Mula | 32,1 | 11,8 | 1 | | | sehr gut | |
| | | | | | | | | |
| 5 | 3. Mula nach Carbonisierung | 7,0 | 2,1 | 5,6 | 30 | 8,1 | | |
| | 3. Niederschlag u. anhaftende Mula | 29,9 | 12,6 | 5,3 | | | akzeptabel | |

**Anmerkung zu Tab.1:** *) Dabei wurde der zunächst experimentell bestimmte auf die Filterkuchenfläche bezogene Filtrationswiderstand $\alpha_H$ [1/m²] umgerechnet auf den auf die trockene Kuchenmasse bezogenen Filtrationswiderstand $\alpha_M$ [m/kg] mittels Division durch die Dichte des trockenen Filterkuchens $\rho_s$ [kg/m³] nach der Formel: $\alpha_M$ [m/kg] = $\alpha_H$ [1/m²] / $\rho_s$ [kg/m³] (siehe Beisp. 1 und Beisp.2) mit Hilfe folgender experimentell bestimmter Messwerte:

$\alpha_H = 1*10^9 *1/m^2$ mit $\rho_s$ 720 kg/m³ in Beisp. 1 und $\alpha_H = 1*10^{10} *1/m^2$ mit $\rho_s$ 720 kg/m³ Beisp.2

| Beispiel | Verfahrensstufe | Konzentrationen in Gew.% Met | $K^+$ Met-Met | | T in°C Carbonisierung | pH Carbonisierung | Filtrierbarkeit | Kuchen - widerstand in m/kg |
|---|---|---|---|---|---|---|---|---|
| | 2. Mula (Ausgangslösung 3 für die Beispiele 6 bis 12) | 7,1 | 3,3 | 4,7 | | | | |
| | 2. Mula nach Aufkonzentrierung um Faktor 1,6 | 11,9 | 5,3 | 7,5 | | | | |
| | | | | | | | | |
| 6 | 3. Mula nach Carbonisierung | 8,2 | 5,8 | 8,3 | 30 | 9 | | |
| | 3. Niederschlag u. anhaftende Mula | 32,7 | 1,9 | 2,6 | | | sehr gut | |
| | | | | | | | | |
| 7 | 3. Mula nach Carbonisierung | 6,4 | 3,6 | 7,6 | 30 | 8,5 | | |
| | 3. Niederschlag u. anhaftende Mula | 28,3 | 8,9 | 2,9 | | | sehr gut | |
| | | | | | | | | |
| 8 (Vgl.) | 3. Mula nach Carbonisierung | 6,2 | 3,1 | 7,3 | 30 | 8,25 | | |
| | 3. Niederschlag u. anhaftende Mula | 22,7 | 9,3 | 6,8 | | | schlecht | |
| | | | | | | | | |
| 9 | 3. Mula nach Carbonisierung | 9,5 | 5,8 | 8,2 | 55 | 8,97 | | |
| | 3. Niederschlag u. anhaftende Mula | 33,1 | 1,8 | 2,6 | | | sehr gut | |
| | | | | | | | | |
| 10 | 3. Mula nach Carbonisierung | 8,5 | 5,9 | 8,3 | 55 | 8,53 | | |
| | 3. Niederschlag u. anhaftende Mula | 34,0 | 1,6 | 2,3 | | | sehr gut | |
| | | | | | | | | |
| 11 | 3. Mula nach Carbonisierung | 8,0 | 4,8 | 8,6 | 50 | 8,47 | | |
| | 3. Niederschlag u. anhaftende Mula | 32,4 | 6,2 | 1,3 | | | gut | |
| | | | | | | | | |
| 12 (Vgl.) | 3. Mula nach Carbonisierung | 5,7 | 2,9 | 7,4 | 25 | 8,27 | | |
| | 3. Niederschlag u. anhaftende Mula | 24,6 | 10,5 | 9,2 | | | sehr schlecht | |

**Kommentierung der Tabelle 1:**

Aus dem Vergleich von Beispiel 1 und 2 in Tabelle 1 wird deutlich, dass das Auftreten von Met-Met im Niederschlag durch eine Erhöhung des pH-Wertes in einen optimalen Bereich hinein weitgehend vermieden werden kann. Auch ein Anheben der Temperatur in der Carbonisierungsstufe oder eine Verringerung des Aufkonzentrierungsfaktors kann verhindern, dass Met-Met ausfällt und so die Filtrierbarkeit verbessern. Eine solche Veränderung dieser Parameter reduziert jedoch auch die Menge an Methionin und Kalium im Niederschlag stark. Bei einer Anhebung des pH-Wertes ist dies weniger stark der Fall. Die Einstellung mit Hilfe des pH-Wertes hat sich daher überraschenderweise als sehr wirksame und gleichzeitig einfache und kostengünstige und damit als die wirtschaftlich vorteilhafteste Lösung herausgestellt.

Ein Vergleich der Beispiele 3 - 5 verdeutlicht den Einfluss des pH-Wertes auf die in Filtrat und Niederschlag gemessenen Konzentrationen. Die Met-Met-Konzentration in der aufkonzentrierten 2. Mutterlauge betrug 5,9 Gew.%. In Beispiel 3 wurde diese Mutterlauge bei 30 °C bis zu einem pH-Wert von 8,9 carbonisiert. Die Met-Met-Konzentration in der resultierenden 3. Mutterlauge stieg dabei auf 6,1 Gew.%. Dies liegt daran, dass die Komponenten Methionin und $KHCO_3$ teilweise ausgefallen waren, denn die Kalium-Konzentration in der 3. Mutterlauge reduzierte sich von 13,1 Gew.% auf 8,2 Gew.% und die Methionin Konzentration von 4,6 Gew.% auf 3,4 Gew.%. Das Ausfallen dieser Komponenten bedingt einen Anstieg der Met-Met-Konzentration, solange Met-Met nicht ebenfalls ausfällt. Im Niederschlag konnten zudem nur 1,2 Gew.% Met-Met gefunden werden. Die Konzentrationsmessungen in Filtrat und Kuchen zeigen, dass hier kein Met-Met ausgefallen war.

In Beispiel 4 ist der gleiche Effekt zu sehen. Die Konzentrationen von Methionin und Kalium sank in der 3. Mutterlauge aufgrund des niedrigeren pH-Wertes von 8,5 weiter ab, die Met-Met-Konzentration stieg weiter auf 6,2Gew.%.

Erst bei der Carbonisierung bis pH 8,1 in Beispiel 5 fiel die Met-Met-Konzentration im Filtrat mit 5,3 Gew.% unter die Anfangskonzentration von 5,9 Gew.%. Zudem beträgt die Met-Met-Konzentration im Niederschlag hier 5,3 Gew.%. Die Filtrierbarkeit des Kuchens verschlechterte sich dabei deutlich: von "sehr gut" auf "akzeptabel".

Ein Vergleich der Beispiele 6, 7 und Vergleichsbeispiel 8 verdeutlicht nochmals den Einfluss des pH-Wertes. In allen drei Beispielen wurde von der gleichen Ausgangslösung (2. Mula nach Aufkonzentration) ausgegangen und diese bei dergleichen Temperatur von 30 °C carbonisiert. Die dabei eingestellten pH-Werte betrugen 9,0, 8,5 und 8,2. Die Löslichkeit und damit die Konzentration der Wertstoffe Kalium und Methionin sowie des Nebenproduktes Met-Met in der 3. Mutterlauge nimmt mit dem pH-Wert ab. In Vergleichsbeispiel 8 ist dabei der ausgefallenen Met-Met-Anteil im Niederschlag mit 6,8 Gew.% schon so groß, dass die Filtrierbarkeit schlecht war im Gegensatz zu den sehr guten Filtrierbarkeiten in den Beispielen 6 und 7.

Ein Vergleich von Beispiel 5 mit Beispiel 8 verdeutlicht den Einfluss des Aufkonzentrierungsfaktors auf die Met-Met-Konzentration der aufkonzentrierten 2. Mutterlauge, die von diesem Faktor direkt abhängig ist. In Beispiel 8 war der Niederschlag bereits bei einem pH-Wert von 8,25 "schlecht" filtrierbar. Dies liegt daran, dass die Met-Met-Konzentration der Ausgangslösung höher war (hier 7,5 Gew.% im Vergleich zu 5,9 Gew.% von Beispiel 5). Dadurch wurde die Löslichkeitsgrenze von Met-Met bereits bei höheren pH-Werten überschritten und es kam demzufolge auch bereits bei höheren pH-Werten zu einem nennenswerten Met-Met-Anteil im Niederschlag, der die Filtrierbarkeit entsprechend herabsetzte. Je höher also die Met-Met Konzentration der Ausgangslösung ist, desto höher liegt auch der pH-Wert, bei dessen Unterschreitung der Met-Met Anteil im Niederschlag nennenswert ansteigt. Je niedriger die Met-Met Konzentration der Ausgangslösung, desto niedriger liegt auch der pH-Wert, der zu einem nennenswerten Met-Met-Anteil im Niederschlag führt. Unterhalb einer Met-Met-Konzentration der Ausgangslösung von 4,5 Gew.% kann die Lösung (bei üblichem Druck und Temperatur) bis zur $CO_2$-Sättigung (d.h. dem, beim jeweiligen Druck, niedrigsten zu erreichenden pH-Wert) carbonisiert werden, ohne dass mit einem nennenswerten Met-Met-Anteil im Niederschlag zu rechnen ist. Da die zuvor erzeugten Mutterlaugen zur 1. bzw. 2. Carbonisierung in der Regel noch einen Gehalt von < 4,5 Gew.% Met-Met aufweisen, tritt hier das Problem der schlechten Filtrierbarkeit nach der Carbonisierung nicht auf. In der 2. Mutterlauge ist Met-Metgegenüber titrierbarem Kalium und Methionin angereichert, da diesen beiden Stoffe durch die Carbonisierung und anschließende Filtration aus der Lösung abgetrennt wurden. Um zu erreichen, dass auch in der 3. Carbonisierung Kaliumhydrogencarbonat und/oder Kaliumcarbonat und Methionin im Niederschlag ausfallen, muss die 2. Mutterlauge zunächst aufkonzentriert werden. In der aufkonzentrierten Ausgangslösung für die 3.Carbonisierung (aufkonzentrierte 2. Mutterlauge), werden daher, im Vergleich zu den vorherigen Carbonisierungen, höhere Met-Met-Konzentrationen erreicht, so dass insbesondere hier die erfinderische Lösung zur Verbesserung der Filtrierbarkeit des Niederschlags zum Tragen kommt.

Ein Vergleich der Beispiele 4, 10 und 11 verdeutlicht den Einfluss der Temperatur. In allen drei Beispielen beträgt der pH-Wert rund 8,5. Die Löslichkeit und damit die Konzentration der Wertstoffe Kalium und Methionin in der 3. Mutterlauge nimmt mit der Temperatur ab. Ein Vergleich der Beispiele 4 und 11 zeigt besonders gut den Einfluss der Temperatur. In beiden Beispielen sind die Ausgangslösungen ähnlich konzentriert und die bei der Carbonisierung eingestellten pH-Werte praktisch identisch, die Temperatur liegt in Beispiel 4 bei 30 °C und im Beispiel 11 bei 50°C. In

Beispiel 4 ist die Met-Met-Konzentration im Niederschlag mit 1 Gew.% jedoch vergleichbar niedrig wie in Beispiel 11 mit 1,3 Gew.%. Mit abnehmender Temperatur fällt mehr Met-Met mit dem Niederschlag aus. Auch hier nehmen die Konzentrationen der Wertstoffe Methionin und Kalium mit der Temperatur ab.

Aus den Messergebnissen in Tab.1 wird jedoch deutlich, dass sich bei einer Carbonisierung bei pH 9,0 gemäß Beispiel 6 nur geringe Mengen von 2,6 Gew.%, Met-Met im Niederschlag befanden. Die Met-Met-Konzentration in der 3. Mutterlauge ist höher als in der konzentrierten 2. Mutterlauge und die Met-Met Konzentration im Niederschlag ist mit 2,6 Gew.% gering. Diese 2,6 Gew.% können auch durch die anhaftende Mutterlauge verursacht worden sein. Bei Carbonisierung bis pH 8,2 ist die Met-Met-Konzentration in der 3. Mutterlauge niedriger als in der konzentrierten 2. Mutterlauge, zudem ist die Met-Met Konzentration im Niederschlag bei 6,4 Gew.%. Hieran ist ablesbar, dass sich Met-Met im Niederschlag befindet.

Der Kuchenwiderstand wurde sowohl für die Carbonisierung bei pH 8,7 ($1{,}2*10^6$ m/kg, siehe Beispiel 1) als auch für die Carbonisierung bei pH 8,2 gemessen (ca. $2{,}3*10^9$ m/kg, siehe Beispiel 2 (Vergleich)). Der Kuchenwiderstand des mit pH 8,2 erhaltenen Niederschlags liegt um den Faktor 2000 höher im Vergleich zum Widerstand des Kuchens bei pH 8,7. Dementsprechend ist die Filtrierbarkeit des Niederschlages bei pH 8,7 sehr gut und damit deutlich besser als die nur noch akzeptable Filtrierbarkeit bei pH 8,2.

Aus den Beispielen kann geschlussfolgert werden, dass die Löslichkeiten der Komponenten Methionin, Kalium (hauptsächlich vorliegend als Kaliumhydrogencarbonat) und Met-Met mit Temperatur und pH-Wert abnehmen. Der Aufkonzentrierungsfaktor sorgt dabei insbesondere für eine große Differenz zwischen den Konzentrationen in der aufkonzentrierten 2. Mutterlauge und in der 3. Mutterlauge und damit für eine größere Masse des Niederschlags (= Wertstoff) und eine kleine Masse der 3. Mutterlauge (= Nebenproduktstrom).

**Beschreibung der Figur:**

Die Figur zeigt ein Schema zum erfindungsgemäßen Methionin-Prozess.

Das Schema umfasst die folgenden Schritte, wobei die Bezugszeichen die in der untenstehenden Tabelle 2 angegebenen Bedeutungen haben.

Tabelle 2: Bezugszeichenliste für Figur 1

| **Bezugszeichen** | **Verfahrensstufe** |
|---|---|
| (1) | Reaktionsschritt Hydrolyse |
| (2) | 1.Carbonisierung |
| | 1.Kristallisation (von 1.Niederschlag) |
| | 1.Fest-Flüssigtrennung (von 1. Niederschlag und 1.Mutterlauge) |
| (3) | 1.Aufkonzentrierung (der 1.Mutterlauge) |
| (4) | Rückführung (eines 1.Teils der aufkonzentrierten 1.Mutterlauge) |
| (5) | 2.Carbonisierung (des 2.Teils der aufkonzentrierten 1.Mutterlauge) |
| | 2.Kristallisation (von 2.Niederschlag) |
| | 2.Fest-Flüssigtrennung (von 2. Niederschlag und 2.Mutterlauge) |
| (6) | Aufkonzentrierung (der 2.Mutterlauge) |
| | 3.Carbonisierung (des 2.Teils der aufkonzentrierten 1.Mutterlauge) |
| | 3.Kristallisation (von 3.Niederschlag) |
| | 3.Fest-Flüssigtrennung (von 2. Niederschlag und 2.Mutterlauge) |

**Patentansprüche**

1. Verfahren zur Gewinnung von Gemischen enthaltend Methionin und Kaliumhydrogencarbonat aus wässrigen Lösungen oder Suspensionen enthaltend titrierbares Kalium in Form von Kaliumhydrogencarbonat und/oder Kaliumcarbonat, Methionin, und
4,5 - 12,0 Gew.% Methionyl-methionin, **dadurch gekennzeichnet, dass** man den eingesetzten Lösungen oder Suspensionen bei einer Temperatur von 15 - 60 °C CO2 zuführt (carbonisiert), so dass ein Gemisch enthaltend

Methionin und Kaliumhydrogencarbonat als Niederschlag ausfällt, der im Mittel max. 6,5 Gew.% Met-Met enthält und dieser von der Mutterlauge abgetrennt wird.

2. Verfahren zur Gewinnung von Gemischen enthaltend Methionin und Kaliumhydrogencarbonat aus wässrigen Lösungen oder Suspensionen enthaltend Methionin, Kaliumhydrogencarbonat und Methionyl-methionin gemäß Anspruch 1, **dadurch gekennzeichnet, dass** man die eingesetzten Lösungen oder Suspensionen gegebenenfalls durch Aufkonzentrieren auf einen Gehalt von

   6,0 - 18,0 Gew.% titrierbares Kalium in Form von Kaliumhydrogencarbonat und/oder Kaliumcarbonat,
   2,5 - 8,0 Gew.% Methionin,
   4,5 - 12,0 Gew.% Methionyl-Methionin bringt und
   bei einer Temperatur von 15 - 60 °C $CO_2$ zuführt, so dass ein Gemisch enthaltend Methionin und Kaliumhydrogencarbonat als Niederschlag ausfällt, der im Mittel max. 6,5 Gew.% Met-Met enthält und dieser von der Mutterlauge abgetrennt wird.

3. Verfahren zur Gewinnung von Gemischen enthaltend Methionin und Kaliumhydrogencarbonat aus wässrigen Lösungen oder Suspensionen enthaltend Methionin, Kaliumhydrogencarbonat und Methionyl-methionin gemäß Anspruch 1 oder 2, **dadurch gekennzeichnet, dass** man die eingesetzten Lösungen oder Suspensionen gegebenenfalls durch Aufkonzentrieren auf einen Gehalt von

   6,0 - 18,0 Gew.% titrierbares Kalium in Form von Kaliumhydrogencarbonat und/oder Kaliumcarbonat,
   2,5 - 8,0 Gew.% Methionin,
   4,5 - 12,0 Gew.% Methionyl-Methionin bringt und
   bei einer Temperatur von 15 - 60 °C solange $CO_2$ zuführt (carbonisiert) bis ein pH-Wert von 7,8 bis 9,5 erreicht wird, gemessen mit einer Glaselektrode bei der eingestellten Temperatur, so dass ein Gemisch enthaltend Methionin und Kaliumhydrogencarbonat als Niederschlag ausfällt, der im Mittel max.6,5 Gew.% Met-Met enthält und dieser von der Mutterlauge abgetrennt wird.

4. Verfahren gemäß einem der Ansprüche 1 bis - 3, **dadurch gekennzeichnet, dass** man bei einer Temperatur von 25 - 55 °C solange $CO_2$ zuführt bis ein pH-Wert von 8,3 bis 9,5 erreicht wird, so dass ein Gemisch enthaltend Methionin und Kaliumhydrogencarbonat als Niederschlag ausfällt, der im Mittel max. 0,01 bis 5,0 Gew.% Met-Met enthält und dieser von der Mutterlauge abgetrennt wird.

5. Verfahren gemäß einem der Ansprüche 1 bis 4, **dadurch gekennzeichnet, dass** man bei einer Temperatur von 25 - 55 °C solange $CO_2$ zuführt bis ein pH-Wert von 8,4 bis 9,5 erreicht wird, so dass ein Gemisch enthaltend Methionin und Kaliumhydrogencarbonat als Niederschlag ausfällt, der im Mittel max. 0,01 bis 3,0 Gew.% Met-Met enthält und dieser von der Mutterlauge abgetrennt wird.

6. Verfahren gemäß einem der Ansprüche 1 bis 5, **dadurch gekennzeichnet, dass** man so lange $CO_2$ zuführt, bis ein pH-Wert von 8,4 bis 9,0 erreicht wird.

7. Verfahren gemäß einem der Ansprüche 1 bis 6, **dadurch gekennzeichnet, dass** der Druck während der $CO_2$-Zufuhr im Bereich 1 - 6 bara liegt.

8. Verfahren gemäß einem der Ansprüche 1 bis 7, **dadurch gekennzeichnet, dass** die Verweilzeit in der $CO_2$-Zufuhr zwischen 20 und 180 Minuten liegt.

9. Verfahren gemäß einem der Ansprüche 1 bis 8, **dadurch gekennzeichnet, dass** als wässrige Lösungen oder Suspensionen enthaltend Methionin, Kaliumhydrogencarbonat und Methionyl-methionin eine Mutterlauge aus der Isolierung von Methionin im Verfahren zur Herstellung von Methionin via alkalische Methioninhydantoinverseifung eingesetzt wird.

10. Verfahren gemäß Anspruch 9, **dadurch gekennzeichnet, dass** es sich bei der Mutterlauge um die Mutterlauge der Filtration nach wiederholter Carbonisierung handelt.

11. Gemisch enthaltend Methionin und Kaliumhydrogencarbonat hergestellt gemäß einem der Ansprüche 1 bis 12.

12. Gemisch gemäß Anspruch 11 enthaltend Methionin, Kaliumhydrogencarbonat und Met-Met, wobei der Met-Met

Gehalt bei max. 6,5 Gew.% liegt.

**13.** Verwendung eines Gemisches nach Anspruch 11 oder 12 zur Herstellung von Methionin.

**14.** Verfahren zur Herstellung von Methionin, das die folgenden Schritte (1) bis (6) umfasst:

(1) einen Reaktionsschritt, umfassend die Hydrolyse von 5-[2-(Methylthio)ethyl]imidazolidin-2,4-dion in Gegenwart einer basischen Kaliumverbindung zu einem Kaliummethioninat und Kaliummethionylmethioninat enthaltenden Hydrolysat;
(2) einen ersten Kristallisationsschritt, der die Einführung von Kohlendioxid (1.Carbonisierung) in das in Schritt (1) erhaltene Hydrolysat umfasst, um eine Suspension enthaltend Methionin, Methionyl-methionin und Kaliumhydrogencarbonat zu erzeugen und dabei Methionin auszufällen, und Trennen der resultierenden Suspension in einen Methionin enthaltenden 1.Niederschlag und eine Methionyl-methionin enthaltende 1.Mutterlauge;
(3) das Konzentrieren der in Schritt (2) erhaltenen 1.Mutterlauge (1.Konzentrationsschritt),
(4) Rückführung eines ersten Teils der konzentrierten 1. Mutterlauge aus (3) in den Reaktionsschritt (1)
(5) einen zweiten Kristallisationsschritt, der das Einleiten von Kohlendioxid in den zweiten Teil der konzentrierten 1. Mutterlauge aus (3) (2.Carbonisierung) umfasst, um Methionin und Kaliumhydrogencarbonat auszufällen, und das Trennen der resultierenden Suspension in einen 2.Niederschlag und eine 2.Mutterlauge,
(6) einen dritten Kristallisationsschritt, der das Konzentrieren der in Schritt (3) erhaltenen 2.Mutterlauge, das Einleiten von Kohlendioxid in die konzentrierte 2.Mutterlauge umfasst, um Methionin und Kaliumhydrogencarbonat auszufällen, und die Trennung der resultierenden Suspension in einen ein Gemisch aus Methionin und Kaliumhydrogencarbonat enthaltenden 3.Niederschlag und eine Methionylmethionin enthaltende 3.Mutterlauge. gemäß einem der Ansprüche 1 bis 9 umfasst, so dass der 3.Niederschlag im Durchschnitt max.6,5 Gew.% Met-Met enthält.

**15.** Verfahren gemäß Anspruch 14, **dadurch gekennzeichnet, dass** der 3. Niederschlag im Durchschnitt max.0,01 bis 5,0 Gew.% Met-Met enthält.

**16.** Verfahren gemäß Anspruch 14 oder 15, **dadurch gekennzeichnet, dass** man den 3. Niederschlag in einen der Schritte (1) bis (4) zurückführt.

**17.** Verfahren gemäß Anspruch 14 und 15, **dadurch gekennzeichnet, dass** man die 1. Mutterlauge und den 3. Niederschlag vereinigt und anschließend in den Reaktionsschritt (1) oder den Konzentrationsschritt (3) zurückführt.

**18.** Verfahren gemäß einem der Ansprüche 14 bis 16, **dadurch gekennzeichnet, dass** man die 3.Mutterlauge entsorgt oder einer weiteren Isolierung von Kaliumhydrogencarbonat und/oder Methionin zuführt.

Figur 1: Verfahrensablauf

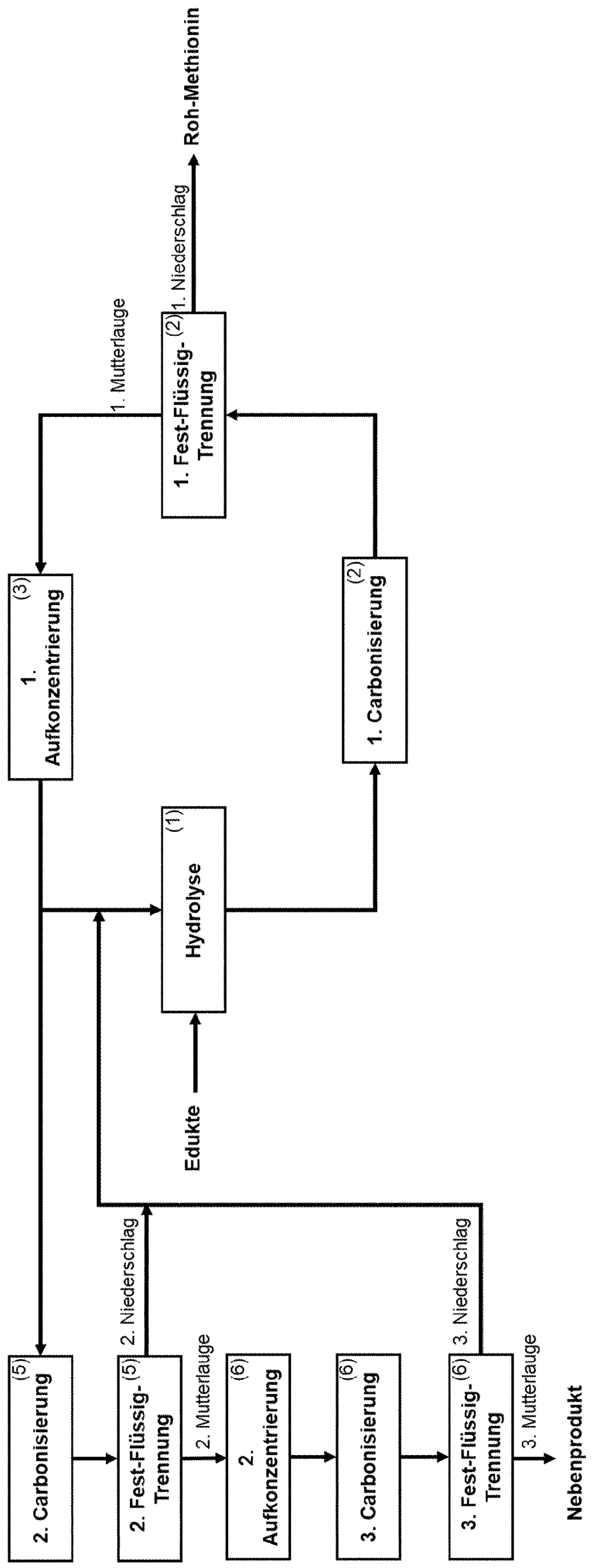

Europäisches Patentamt
European Patent Office
Office européen des brevets

# EUROPÄISCHER RECHERCHENBERICHT

Nummer der Anmeldung
EP 22 17 9609

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 2 133 328 A2 (SUMITOMO CHEMICAL) 16. Dezember 2009 (2009-12-16) * Absätze [0006], [0028], [0039] - [0043]; Ansprüche 1, 2 * ----- | 1-18 | INV. C07C319/28 C07C319/20 C07C323/58 C07K5/062 |
| A,D | EP 2 186 798 A1 (SUMITOMO CHEMICAL) 19. Mai 2010 (2010-05-19) * Absätze [0006], [0020] - [0021], [0039] - [0043]; Ansprüche 1, 2 * ----- | 1-18 | |

RECHERCHIERTE SACHGEBIETE (IPC)
C07C
C07K

Der vorliegende Recherchenbericht wurde für alle Patentansprüche erstellt

1

| Recherchenort | Abschlußdatum der Recherche | Prüfer |
|---|---|---|
| Den Haag | 7. Dezember 2022 | English, Russell |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 22 17 9609

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-12-2022

| Im Recherchenbericht angeführtes Patentdokument | Datum der Veröffentlichung | Mitglied(er) der Patentfamilie | Datum der Veröffentlichung |
|---|---|---|---|
| EP 2133328 A2 | 16-12-2009 | CN 101602700 A | 16-12-2009 |
| | | EP 2133328 A2 | 16-12-2009 |
| | | JP 2009292796 A | 17-12-2009 |
| | | SG 158027 A1 | 29-01-2010 |
| | | US 2010004486 A1 | 07-01-2010 |
| EP 2186798 A1 | 19-05-2010 | CN 101735126 A | 16-06-2010 |
| | | EP 2186798 A1 | 19-05-2010 |
| | | JP 2010111640 A | 20-05-2010 |
| | | SG 161197 A1 | 27-05-2010 |
| | | US 2010121102 A1 | 13-05-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 780370 A2
- DE 2421167 A1
- EP 839804 A2
- EP 1760074 A1
- EP 2133328 A2
- EP 2186798 A1
- EP 2133328 A
- EP 2186797 A1
- EP 2133329 A2
- EP 2133329 A
- EP 1840119 A2